(19) Europäisches Patentamt
European Patent Office
Office européen des brevets

(11) Numéro de publication: **0 066 483**
**B1**

# (12) FASCICULE DE BREVET EUROPEEN

(45) Date de publication du fascicule du brevet: **21.11.84**

(51) Int. Cl.³: **C 07 C 120/00**, C 07 C 121/66, B 01 J 27/12

(21) Numéro de dépôt: **82400803.1**

(22) Date de dépôt: **03.05.82**

(54) **Procédé de préparation de nitriles aromatiques ou aliphatiques.**

(30) Priorité: **15.05.81 FR 8109695**

(43) Date de publication de la demande: **08.12.82 Bulletin 82/49**

(45) Mention de la délivrance du brevet: **21.11.84 Bulletin 84/47**

(84) *Etats contractants désignés:* **AT BE CH DE FR GB IT LI LU NL SE**

(56) Documents cités: **FR - A - 1 250 165**

**Le dossier contient des informations techniques présentées postérieurement au dépôt de la demande et ne figurant pas dans le présent fascicule.**

(73) Titulaire: **RHONE-POULENC SPECIALITES CHIMIQUES, "Les Miroirs" 18, Avenue d'Alsace, F-92400 Courbevoie (FR)**

(72) Inventeur: **Jacques, Roland, 579, Montée de Silhol, F-30100 Ales (FR)**
Inventeur: **Reppelin, Michel, 10, Chemin Neuf, F-69660 Collonges-au-Mont-d'Or (FR)**
Inventeur: **Seigneurin, Laurent, 3, avenue du Parc, F-30340 Salindres (FR)**

(74) Mandataire: **Cazes, Jean-Marie et al, RHONE-POULENC RECHERCHES Service Brevets Chimie et Polymères 25, quai Paul Doumer, F-92408 Courbevoie *Cedex* (FR)**

**EP 0 066 483 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de préparation de nitriles aromatiques ou aliphatiques.

Elle concerne plus particulièrement la prèparation de nitriles de formule générale:

Ar—A—CN (I)

où Ar représente un radical benzénique et où A représente un radical hydrocarboné contenant de 1 à 6 atomes de carbone, à partir des formamides ou formanilides de formule générale:

Ar—A—NHCHO (II)

ou des amides de formule générale:

$Ar—A—CONH_2$ (III)

dans lesquelles Ar et A ont la signification précédente.

On connait dans l'art antérieur le brevet français 1 250 165 qui décrit la préparation de nitriles à partir de composés du type (II) par réaction de ces derniers à une température comprise entre 460 et 560° C en phase gazeuse au contact, comme catalyseurs, d'acide silicique actif ou de silicates renfermant un oxyde métallique.

Les expériences effectuées par la demanderesse avec des catalyseurs du type de ceux décrits dans le brevet français précité montrent que la sélectivité obtenue n'est pas suffisante pour une exploitation industrielle optimale du procédé. De plus, lorsque des nitriles de formule I dont le radical Ar comporte un substituant fluoré sont recherchés, on assiste à la formation de produits secondaires lourds ce qui diminue la durée de vie du catalyseur par encrassement de la surface et on observe des réactions de défluoration ce qui donne naissance à des composés très difficilement séparables du produit recherché.

Un objet de la présente invention est de fournir un procédé de préparation de nitriles aromatiques ou aliphatiques palliant les inconvénients précités de l'art antérieur.

Un autre objet de la présente invention est de fournir un procédé de préparation de nitriles aromatiques ou aliphatiques à partir d'amides de formule (III).

La présente invention a donc pour objet un procédé de préparation de nitriles aromatiques ou aliphatiques de formule:

Ar—A—CN (I)

dans laquelle Ar représente un radical benzénique et A représente un lien valentiel ou un radical hydrocarboné contenant de 1 à 6 atomes de carbone caractérisé en ce que l'on porte à une température comprise entre 450° C et 550° C un formanilide ou formamide de formule:

Ar—A—NHCHO (II)

ou un amide de formule:

$Ar—A—CONH_2$ (III)

où Ar et A ont la signification précédente en présence d'un catalyseur préparé par imprégnation à l'aide d'une solution aqueuse d'acide fluorhydrique, ayant une concentration en HF inférieure à environ 5% en poids, d'une silice obtenue à partir de silicate de sodium et d'acide sulfurique, le rapport pondéral de l'acide fluorhydrique contenu dans la solution aqueuse à la silice étant inférieur à environ 5%, puis séchage.

On entend, au sens de la présente invention, par imprégnation de la silice, la mise en contact de cette dernière avec une solution aqueuse d'acide fluorhydrique.

Selon un mode de réalisation particulier, on opère par trempage de la silice dans la solution aqueuse d'acide fluorhydrique.

Selon un autre mode de réalisation particulier, on opère par pulvérisation de la solution aqueuse d'acide fluorhydrique sur la silice.

Dans l'un et l'autre cas, la quantité de solution d'acide fluorhydrique introduite dans la silice sera environ égale au volume poreux total de la silice traitée.

La demanderesse a constaté que pour une bonne mise en oeuvre de l'invention, il était préférable d'imprégner une silice ayant une surface spécifique comprise entre 200 et 350 $m^2/g$, un volume poreux total compris entre 50 et 100 $cm^3/g$, un diamètre moyen des pores compris entre et 110 Å; un pH d'échange inférieur à environ 7 et une teneur en sodium exprimée en $Na_2O$ inférieure à environ 1% en

poids par rapport à la silice.

Selon un autre mode de réalisation préférentiel de l'invention, l'imprégnation est réalisée à l'aide d'une solution aqueuse d'acide fluorhydrique ayant une concentration comprise entre 0,04 et 4% en poids.

L'imprégnation est de préférence réalisée à température ambiante sous pression atmosphérique.

Le séchage est de préférence réalisé à une température comprise entre 150 et 600° C pendant 1 à 24 heures.

La silice comprendra avantageusement après séchage entre 0,3 et 3% en poids de flour introduit par imprégnation.

Les silices à imprégner sont obtenues classiquement par précipitation de silicate de sodium à l'aide d'acide sulfurique (voir par exemple le brevet français publié sous le numéro 2 093 196).

Au sens de la présente invention, on entend par radical benzénique (Ar) le radical phényle et les radicaux phényle comportant un ou plusieurs substituants. On peut citer comme exemples de tels substituants les radicaux alkyle et alkoxy ayant de 1 à 6 atomes de carbone, les radicaux phényle et phénoxy et les radicaux F, $CF_3$, $OCF_3$; $SCF_3$, OH, Cl, Br, CN.

Le procédé selon l'invention est plus particulièrement adapté à la mise en oeuvre de composés de formule II ou III dont le radical phényle comporte un ou plusieurs substituants fluorés F, $CF_3$, $OCF_3$ ou $SCF_3$. En effet, dans ce cas, on obtient une faible quantité de produits résultant des réactions de défluoration.

Le procédé est encore plus adapté à la mise en oeuvre des composés de formule II comportant un radical fluoré. Parmi ces derniers, on peut citer le métatrifluorométhylformanilide

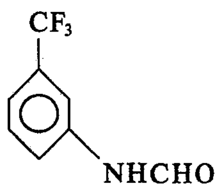

et le métatrifluorométhylbenzylformamide de formule:

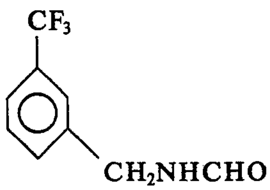

qui permettent d'obtenir respectivement le métatrifluorométhylbenzonitrile et le métatrifluorométhylphénylacétonitrile qui sont de très intéressants intermédiaires de synthèse de composés à activité phytosanitaire ou pharmaceutique.

On peut citer comme autres exemples de formule II pouvant être mis en oeuvre le procédé de l'invention, les composés suivants:

formanilide, trifluorométhyl-3 formanilide, trifluorométhyl-4 formanilide, méthoxy-4 formanilide, hydroxy-4 formanilide, fluoro-2 formanilide, fluoro-3 formanilide, fluoro-4 formanilide, chloro-2 formanilide, chloro-3 formanilide, chloro-4 formanilide, chloro-2 trifluorométhyl-5 formanilide, trifluorométhyl-3 chloro-4 formanilide, phénoxy-3 formanilide, bistrifluorométhyl-3,5 formanilide, dichloro-2,6 formanilide, difluoro-2,6 formanilide, difluoro-2,4 formanilide, trifluorméthylthio-3 formanilide, benzylformamide, trifluorométhyl-3 benzyl formamide, trifluorométhyl-4 benzylformamide, fluoro-4 benzylformamide, chloro-4 benzylformamide, fluoro-2 benzylformamide, chloro-2 benzylformamide, trifluorométhoxy-2 benzylformamide, trifluorméthoxy-4 benzylformamide, trifluorméthylthio-2 benzylformamide, trifluorométhylthio-4 benzylformamide, fluoro-2 méthyl-5 benzylformamide, fluoro-3 méthyl-6 benzylformamide, chloro-2 trifluorométhoxy-5 benzylformamide, trifluorométhoxy-2 chloro-5 benzylformamide, difluoro-2,5 benzylformamide, difluoro-2,4 benzylformamide.

Des exemples non limitatifs de composés III sont les suivants:

trifluorométhyl-3 benzamide, trifluorométhyl-4 benzamide, fluoro-2 benzamide, fluoro-3 benzamide, fluoro-4 benzamide, trifluorométhyl-3 phénylacétamide, fluoro-4 phénylacétamide, trifluorométhoxy-4 phénylacétamide.

On peut ainsi préparer selon le procédé de l'invention, les composés de formule I suivants:

benzonitrile, trifluorométhyl-3 benzonitrile, trifluorométhyl-4 benzonitrile, méthoxy-4 benzonitrile, hydroxy-4 benzonitrile, fluoro-2 benzonitrile, fluoro-3 benzonitrile, fluoro-4 benzonitrile, chloro-2 benzonitrile, chloro-3 benzonitrile, chloro-4 benzonitrile, chloro-2 trifluorméthyl-5 benzonitrile, trifluorométhyl-3 chloro-4 benzonitrile, phénoxy-3 benzonitrile, bis-trifluorométhyl-3,5 benzonitrile, dichloro-2,6 benzonitrile, difluoro-2,6 benzonitrile, difluoro-2,4 benzonitrile, trifluorométhylthio-3 benzonitrile, trifluorométhoxy-4 benzonitrile, phénylacétonitrile, trifluorméthyl-3 phénylacétonitrile, trifluorométhyl-4 phénylacétonitrile, fluoro-4 phénylacétonitrile, chloro-4 phénylacétonitrile, fluoro-2 phénylacétonitrile, chloro-2 phénylacétonitrile, trifluorométhoxy-2 phénylacétonitrile, trifluorométhoxy-4 phénylacétonitrile, trifluorométhylthio-2 phénylacétonitrile,trifluorométhylthio-4 phénylacétonitrile, fluoro-2 méthyl-5 phénylacétonitrile, fluoro-3 méthyl-6 phénylacétonitrile, chloro-2 trifluorométhoxy-5 phénylacétonitrile, trifluorméthoxy-2 chloro-5 phénylacétonitrile, difluoro-2,5 phénylacétonitrile, difluoro-2,4 phénylacétonitrile.

Selon une manière avantageuse, mais non obligatoire, de mettre en oeuvre l'invention, on opère en présence d'un diluant gazeux inerte constitué de préférence par l'azote et/ou le $CO_2$ et/ou l'acétonitrile.

On préfère utiliser l'acétonitrile en quantité telle que le pourcentage molaire de composé de formule II ou III dans l'acétonitrile soit compris entre 2 et 20 et de préférence entre 5 et 10.

La température sera, préférentiellement comprise entre 510 et 530° C quand on mettra en oeuvre un composé de formule II entre 450 et 480° C quand on mettra en oeuvre un composé de formule III.

On opère généralement à la pression atmosphérique bien que des pressions supérieures ou inférieures à la pression atmosphérique ne soient pas exclues du domaine de l'invention.

On met en oeuvre le procédé selon l'invention en faisant passer, de préférence, entre 0,2 et 4 moles de composé II ou III par heure et par litre de catalyseur.

Les composés III peuvent être préparés par toute technique connue de l'homme de l'art.

La préparation des composés II quand A est un lien valentiel se fait, d'une façon bien connue dans l'art antérieur, par réaction de l'aniline correspondante avec l'acide formique.

Dans le cas où A est un radical hydrocarboné comme $-CH_2-$ les composés II peuvent être obtenus par réaction à 0—100° C en présence d'acide fluorhydrique entre le dérivé benzénique correspondant ArH et l'hydroxyméthylformamide $OH-CH_2-NHCHO$. Le rapport de l'acide fluorhydrique à ArH est compris entre 5 et 50 et celui de ArH à $OH-CH_2-NHCHO$ est compris entre 0,5 et 2. Cette préparation fait l'objet d'une demande de brevet au nom de la demanderesse.

D'autres caractéristiques et avantages de la présente invention apparaitront plus clairement à la lecture des exemples qui vont suivre. Ces exemples ne sauraient en aucune manière être considérés comme une limitation de l'invention.

## Exemple 1

### a) Préparation de la silice

α) On prépare une solution acide contenant 1 kg d'acide sulfurique à 98% et 1 kg d'eau puis une deuxième solution de silicate de soude de densité 1,185 ($SiO2/Na20 = 3,3$).

La solution de silicate est versée dans la solution d'acide sulfurique vigoureusement agitée tout en maintenant la température entre 15 et 20° C. On s'arrête lorsqu'on a versé 1900 g de silicate de soude. La solution gélifie en quelques minutes. L'hydrogel est concassé puis lavé par un courant d'eau à pH compris entre 6 et 7 pendant 24 heures. Les solides obtenus sont séchés à 200° C pendant 24 heures.

Les caractéristiques de la silice obtenue sont les suivantes:

| | |
|---|---|
| — surface spécifique | 420 $m^2/g$ |
| — volume poreux total | 75 $cm^3/100$ g |
| — teneur en Na20 | 900 ppm |
| — pH d'échange | 6,1 |
| — diamètre des pores | 35 Å |

β) 2250 g de cette silice sont introduits dans un réacteur contenant 3 litres d'eau. La silice est totalement immergée. L'ensemble est chauffé jusqu'à obtention dans le réacteur d'une pression relative de 0,7 bars que l'on maintient 3 heures.

Un échantillon de la silice ainsi traitée, après séchage pendant 24 heures à 200° C a les carractéristiques suivantes:

| | |
|---|---|
| — surface spécifique | 320 $m^2/g$ |
| — volume poreux total | 75 $cm^3/100$ g |
| — teneur en Na 20 | 850 ppm |

— pH d'échange 6
— diamètre des pores 85 Å

b) Imprégnation de la silice à l'aide d'acide fluorhydrique en solution aqueuse

On ouvre le réacteur et on ajoute 90 $cm^3$ d'une solution aqueuse à 45% d'HF. On fait circuler la solution aqueuse à travers le lit de gel de silice. On sèche ensuite le gel de silice 24 heures à 200° C.

Les caractéristiques de la silice obtenue sont les suivantes:

— surface spécifique 294 $m^2/g$
— volume poreux total 75 $cm^3/100$ g
— teneur en Na20 750 ppm
— pH d'échange 2,2
— diamètre des pores 90 Å
— teneur en fluor 1,6%

c) Préparation de métatrifluorométhylbenzonitrile à partir de métatrifluorométhylformanilide

Dans un réacteur tubulaire en acier inoxydable d'un litre rempli du catalyseur préparé selon a et b, on introduit en continu en 150 heures, un mélange de 16,1 kg de métatrifluorométhylformanilide et 64,6 kg d'acétonitrile.

La température réactionnelle est maintenue à 530° C tout au long du lit catalytique.

On récupère après distillation du solvant et de l'eau formée:

12 kg de métatrifluorométhylbenzonitrile
1,25 kg de métatrifluorométhylaniline
0,2 kg duformanilide non transformé
1,2 kg de produits lourds

La sélectivité en nitrile est de 91,6%. La teneur en fluorures dans le mélange brut sortant du réacteur est de 350 ppm.

Dans cet exemple, comme dans les exemples ultérieurs, la sélectivité est définie comme étant le rapport du nitrile formé au formanilide ayant réagi l'exclusion du formanilide transformé en aniline correspondante puisque cette dernière, dans un procédé industriel peut être quantitativement par l'acide formique en formanilide de départ alors recyclé.

Exemple 2

2250 g de la silice préparée suivant l'exemple la $\alpha$ sont introduits dans un réacteur contenant trois litres d'eau. La silice est totalement immergée. L'ensemble est chauffé jusqu'à obtenir dans le réacteur une pression relative à 1 bar. Cette pression est maintenue pendant 3 heures. La silice est séchée 3 heures à 200° C.

Cette silice ainsi traitée a les caractéristiques suivantes:

— surface spécifique 235 $m^2/g$
— volume poreux total 80 $cm^3/100$ g
— teneur en Na20 1000 ppm
— pH d'échange 6
— diamètre des pores 90 Å

b) Imprégnation à l'aide d'acide fluorhydrique en solution aqueuse

500 g de cette silice sont introduits dans un bol tournant. On pulvérise sur les grains en rotation, 550 g d'une solution aqueuse contenant 10 g d'HF. Ces grains sont ensuite séchés à 200° C pendant 24 heures.

La silice obtenue a les caractéristiques suivantes:

— surface spécifique 220 $m^2/g$
— volume poreux total 80 $cm^3/100$ g
— teneur en Na20 1000 ppm

- pH d'échange 2,8
- diamètre des pores 95 Å
- teneur en F 1,7%

c) Préparation de métatrifluorométhylphényl acétonitrile à partir de métatrifluorométhylbenzylformamide

En opérant comme dans l'exemple 1c, on introduit en continu en 150 heures, un mélange de 17,9 kg de métatrifluorométhylbenzylformamide et 71,6 kg d'acétonitrile.

La température réactionnelle est maintenue à 530° C tout au long du lit catalytique.

On récupère après distillation du solvant et de l'eau formée:

14,3 kg de métatrifluorométhylphénylacétonitrile
1,1 kg de métatrifluorométhylbenzylamine
0,55 kg du benzylformamide non transformé
0,33 kg de produits lourds

La sélectivité en nitrile est de 98%.
La teneur en fluorure est de 350 ppm.

Exemple 3

a) Imprégnation à l'aide d'acide fluorhydrique en solution aqueuse d'une silice obtenue selon 2a

De la silice préparée selon l'exemple 2a est introduite dans un bol tournant. On pulvérise sur les grains en rotation 350 g d'une solution aqueuse contenant 20 g d'HF. Les grains sont ensuite séchés à 200° C pendant 24 heures.

La silice obtenue présente les caractéristiques suivantes:

- surface spécifique 210 $m^2/g$
- volume poreux total 80 $cm^3/100$ g
- teneur en Na20 1000 ppm
- pH d'échange 2,7
- diamètre des pores 100 Å
- teneur en F 1,8%

b) Préparation de parafluorobenzonitrile à partir de parafluoroformanilide

En opérant comme dans l'exemple 1c on introduit en continu en 300 h. à 520° C, 45 kg de parafluoroformanilide et 180 kg d'acétonitrile.

Après distillation du solvant et de l'eau formée, on récupère:

32,8 kg de parafluorobenzonitrile
4,3 kg de parafluoroaniline
0,5 kg du formanilide non transformé
2 kg de produits lourds

La sélectivité en nitrile est de 95%.
La teneur en fluorures est de 60 ppm.

**Revendications**

1. Procédé de préparation de nitriles aromatiques ou aliphatiques de formule:

Ar—A—CN (I)

dans laquelle Ar représente un radical benzénique et A représente un lien valentiel ou un radical hydrocarboné contenant de 1 à 6 atomes de carbone caractérisé en ce que l'on porte à une température comprise entre 450° C et 550° C un formamide ou formanilide de formule:

Ar—A—NHCHO (II)

ou un amide de formule:

Ar—A—$CONH_2$ (III)

où Ar et A ont la signification précédente en présence d'un catalyseur préparé par imprégnation à l'aide d'une solution aqueuse diluée d'acide fluorhydrique ayant une concentration en HF inférieure à 5% en poids environ, d'une silice obtenue à partir de silicate de sodium et d'acide sulfurique, le rapport pondéral de l'acide fluorhydrique contenu dans la solution aqueuse à la silice étant inférieur à environ 5%, puis séchage.

2. Procédé selon la revendication 1 caractérisé en ce qu'on effectue l'imprégnation par trempage de la silice dans la solution aqueuse d'acide fluorhydrique.

3. Procédé selon la revendication 1 caractérisé en ce qu'on effectue l'imprégnation par pulvérisation de la solution aqueuse d'acide fluorhydrique sur la silice.

4. Procédé selon l'une quelconque des revendications précédentes caractérisé en ce que la silice à imprégner a une surface spécifique comprise entre 200 et 350 $m^2/g$, un volume poreux total compris entre 50 et 100 $cm^3/g$, un diamètre moyen des pores compris entre 70 et 110 Å, un pH d'échange inférieur à environ 7 et une teneur en sodium exprimé en $Na_2O$ inférieure à environ 1% en poids par rapport à la silice.

5.Procédé selon l'une quelconque des revendications précédentes caractérisé en ce que l'imprégnation est réalisée à l'aide d'une solution aqueuse d'acide fluorhydrique ayant une concentration comprise entre 0,04 et 4% en poids.

6. Procédé selon l'une quelconque des revendications précédentes caractérisé en ce que l'imprégnation est réalisée à température ambiante et sous pression atmosphérique.

7. Procédé selon l'une quelconque des revendications précédentes caractérisé en ce que le séchage est effectué à une température comprise entre 150° C et 600° C pendant 1 à 24 h environ.

8. Procédé selon l'une quelconque des revendications précédentes caractérisé en ce que l'on met en oeuvre un composé de formule II ou III dans laquelle Ar représente un radical phényle ou un radical phényle comportant un ou plusieurs substituants choisi parmi le groupe comprenant les radicaux alkyle et alkoxy ayant de 1 à 6 atomes de carbone, phényle et phénoxy, F, $CF_3$, $OCF_3$, $SCF_3$, OH, Cl, Br, CN.

9. Procédé selon la revendication 8 caractérisé en ce que l'on met en oeuvre un composé de formule II ou III dans laquelle Ar représente un radical phényle comportant un ou plusieurs constituants choisi parmi le groupe comprenant F, $CF_3$, $OCF_3$, $SCF_3$.

10. Procédé selon la revendication 9 caractérisé en ce que l'on met en oeuvre le métatrifluorométhylformanilide de formule:

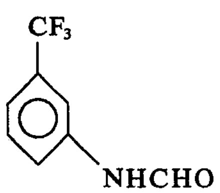

11. Procédé selon la revendication 9 caractérisé en ce que l'on met en oeuvre le métatrifluorométhylbenzylformamide de formule:

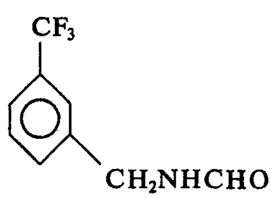

12. Procédé selon l'une quelconque des revendications précédentes caractérisé en ce que l'on fait réagir le composé de formule II ou III en présence d'un diluant gazeux inerte.

13. Procédé selon la revendication 12 caractérisé en ce que le diluant gazeux inerte est constitué par de l'azote et/ou du $CO_2$ et/ou de l'acétonitrile.

14. Procédé selon la revendication 13 caractérisé en ce que le diluant gazeux est l'acétonitrile utilisé en quantité telle que le pourcentage molaire du composé de formule II ou III dans l'acétonitrile soit compris entre 2 et 20.

**Patentansprüche**

1. Verfahren zur Herstellung von aromatischen oder aliphatischen Nitrilen der Formel:

Ar—A—CN (I)

in der Ar einen Benzolrest bedeutet und A eine Valenzbindung oder einen Kohlenwasserstoffrest mit 1 bis 6 Kohlenstoffatomen darstellt, dadurch gekennzeichnet, daß man ein Formamid oder Formanilid der Formel:

Ar—A—NHCHO (II)

oder ein Amid der Formel:

Ar—A—$CONH_2$ (III)

worin Ar und A die oben angegebene Bedeutung haben, in Gegenwart eines Katalysators, der durch Imprägnierung eines aus Natriumsilikat und Schwefelsäure erhaltenen Siliciumdioxids mit einer verdünnten wäßrigen Fluorwasserstoffsäurelösung mit einer Konzentration von weniger als etwa 5 Gew.-%, wobei das Gewichtsverhältnis zwischen der Fluorwasserstoffsäure in der wäßrigen Lösung und dem Siliciumdioxid unter etwa 5% liegt, und durch anschließende Trocknung hergestellt wird, auf eine Temperatur zwischen 450 und 500° C bringt.

2. Verfahren nach Anspruch 1, dadurch gekennzeichnet, daß die Imprägnierung durch Tauchen des Siliciumdioxids in der wäßrigen Fluorwasserstoffsäurelösung durchgeführt wird.

3. Verfahren nach Anspruch 1, dadurch gekennzeichnet, daß die Imprägnierung durch Versprühen der wäßrigen Fluorwasserstoffsäurelösung auf das Siliciumdioxid durchgeführt wird.

4. Verfahren nach einem der vorangehenden Ansprüche, dadurch gekennzeichnet, daß das zu imprägnierende Siliciumdioxid eine spezifische Oberfläche zwischen 200 und 350 $m^2/g$, ein Gesamtporenvolumen zwischen 50 und 100 $cm^3/g$, einen mittleren Porendurchmesser zwischen 70 und 110 Å, einen Austausch-pH unter etwa 7 und einen Natriumgehalt, ausgedrückt als $Na_2O$, von unter etwa 1 Gew.-%, bezogen auf das Siliciumdioxid, hat.

5. Verfahren nach einem der vorangehenden Ansprüche, dadurch gekennzeichnet, daß die Imprägnierung mit Hilfe einer wäßrigen Fluorwasserstofflösung mit einer Konzentration zwischen 0,04 und 4 Gew.-% durchgeführt wird.

6. Verfahren nach einem der vorangehenden Ansprüche, dadurch gekennzeichnet, daß die Imprägnierung bei Umgebungstemperatur und unter Atmosphärendruck durchgeführt wird.

7. Verfahren nach einem der vorangehenden Ansprüche, dadurch gekennzeichnet, daß die Trocknung bei einer Temperatur zwischen 150° und 660° C zwischen ca. 1 und 24 Stunden durchgeführt wird.

8. Verfahren nach einem der vorangehenden Ansprüche, dadurch gekennzeichnet, daß man eine Verbindung der Formel II oder III einsetzt, in der Ar ein Phenylrest oder ein Phenylrest mit einem oder mehreren Substituenten ist, die aus der Gruppe gewählt sind, die die Alkyl- und Alkoxyreste mit 1 bis 6 Kohlenstoffatomen, die Phenyl- und Phenoxyreste sowie die F-, $CF_3$-, $OCF_3$-, $SCF_3$-, OH-, CL-, BR-, CN-Reste.

9. Verfahren nach Anspruch 8, dadurch gekennzeichnet, daß man eine Verbindung der Formel II oder III einsetzt, in der Ar ein Phenylrest ist, der einen oder mehrere Substituenten aus der Gruppe von F, $CF_3$, $OCF_3$, $SCF_3$ trägt.

10. Verfahren nach Anspruch 9, dadurch gekennzeichnet, daß man Metatrifluormethylformanilid der folgenden Formel einsetzt:

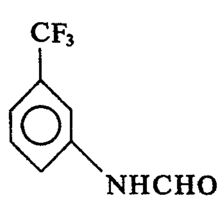

11. Verfahren nach Anspruch 9, dadurch gekennzeichnet, daß man das Metatrifluormethylbenzylformamid der folgenden Formel einsetzt:

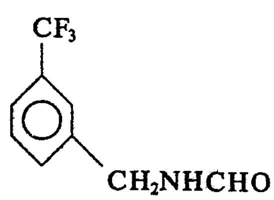

12. Verfahren nach einem der vorangehenden Ansprüche, dadurch gekennzeichnet, daß man die Verbindung der Formel II oder III in Gegenwart eines inerten gasförmigen Verdünnungsmittels reagieren läßt.

13. Verfahren nach Anspruch 12, dadurch gekennzeichnet, daß das inerte gasförmige Verdünnungsmittel Stickstoff und/oder $CO_2$ und/oder Acetonitril ist.

14. Verfahren nach Anspruch 13, dadurch gekennzeichnet, daß das gasförmige Verdünnungsmittel Acetonitril ist und in einer derartigen Menge eingesetzt wird, daß die molare Konzentration der Verbindung der Formel II oder III in dem Acetonitril zwischen 2 und 20 Prozent beträgt.

**Claims**

1. Process for the preparation of aromatic or aliphatic nitriles of the formula

$$Ar-A-CN \quad (I)$$

in which Ar presents a benzene radical and A represents a valency bond or a hydrocarbon radical containing from 1 to 6 carbon atoms, characterised in that a formamide or formanilide of the formula

$$Ar-A-NHCHO \quad (II)$$

or an amide of the formula

$$Ar-A-CONH_2 \quad (III)$$

where Ar and A have the above meaning is heated to a temperature of between 450° C and 550° C in the presence of a catalyst prepared by impregnating a silica, obtained from sodium silicate and sulphuric acid, with a dilute aqueous hydrofluoric acid solution having an HF concentration of less than about 5% by weight, the weight ratio of hydrofluoric acid contained in the aqueous solution to the silica being less than about 5%, and then drying the catalyst.

2. Process according to Claim 1, characterised in that the impregnation is carried out by steeping the silica in the aqueous hydrofluoric acid solution.

3. Process according to Claim 1, characterised in that the impregnation is carried out by spraying the aqueous hydrofluoric acid solution onto the silica.

4. Process according to any one of the preceding claims, characterised in that the silica to be impregnated has a specific surface area of between 200 and 350 $m^2/g$, a total pore volume of between 50 and 100 $cm^3/g$, a mean pore diameter of between 70 and 110 Å, an exchange pH of less than about 7 and a sodium content, expressed as $Na_2O$, of less than about 1% by weight relative to the silica.

5. Process according to any one of the preceding claims, characterised in that the impregnation is carried out with an aqueous hydrofluoric acid solution having a concentration of between 0,04 and 4% by weight.

6. Process according to any one of the preceding claims, characterised in that the impregnation is carried out at ambient temperature and under atmospheric pressure.

7. Process according to any one of the preceding claims, characterised in that the drying is carried out at a temperature of between 150° C and 600° C for about 1 to 24 hours.

8. Process according to any one of the preceding claims, characterised in that a compound of the formula II or III is employed, in which Ar represents a phenyl radical, or a phenyl radical containing one or more substituents selected from among the group comprising alkyl and alkoxy radicals having from 1 to 6 carbon atoms, phenyl and phenoxy, F, $CF_3$, $OCF_3$, $SCF_3$, OH, Cl, Br and CN radicals.

9. Process according to Claim 8, characterised in that a compound of the formula II or III is employed, in which Ar represents a phenyl radical containing one or more substituents chosen from the group comprising F, $CF_3$, $OCF_3$ and $SCF_3$.

10. Process according to Claim 9, characterised in that meta-trifluoromethylformanilide of the formula

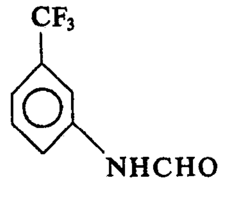

is employed.

11. Process according to Claim 9, characterised in that meta-trifluoromethylbenzylformamide of the formula

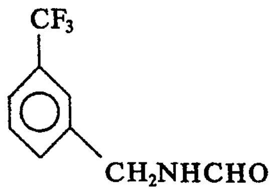

is employed.

12. Process according to any one of the preceding claims, characterised in that the compound of the formula II or III is reacted in the presence of an inert gaseous diluent.

13. Process according to Claim 12, characterised in that the inert gaseous diluent consists of nitrogen and/or $CO_2$ and/or acetonitrile.

14. Process according to Claim 13, characterised in that the gaseous diluent is acetonitrile used in such an amount that the molar percentage of the compound of the formula II or III in the acetonitrile is between 2 and 20.